(19) Europäisches Patentamt
European Patent Office
Office européen des brevets

(11) Publication number: 0 222 670 B1

# (12) EUROPEAN PATENT SPECIFICATION

(45) Date of publication of patent specification: 16.01.91 Bulletin 91/03

(51) Int. Cl.$^5$: A61K 7/06

(21) Application number: 86402514.3

(22) Date of filing: 12.11.86

## (54) Dissolved composition of (benzo-1,2,4-thiadiazine)-1,1-dioxide.

The file contains technical information submitted after the application was filed and not included in this specification

(30) Priority: 15.11.85 JP 256167/85
14.10.86 JP 243968/86

(43) Date of publication of application: 20.05.87 Bulletin 87/21

(45) Publication of the grant of the patent: 16.01.91 Bulletin 91/03

(84) Designated Contracting States: CH DE FR GB IT LI

(56) References cited:
EP-A- 0 027 655
CH-A- 558 376
CH-A- 617 692
US-A- 3 361 816
US-A- 4 022 894
THE MERCK INDEX, 10th edition, MERCK & CO., INC., Rahway, N.J., US; 1983, page 435, abstract no. 2975: "Diazoxide"

(73) Proprietor: SHISEIDO COMPANY LIMITED
7-5-5 Ginza
Chuo-ku Tokyo (JP)

(72) Inventor: Suzuki, Takashi
Shiseido Laboratories 1050 Nippa-Cho, Kohoku-Ku
Yokohama-Shi Kanagawa (JP)
Inventor: Uzuka, Makoto
Shiseido Laboratories 1050 Nippa-Cho, Kohoku-Ku
Yokohama-Shi Kanagawa (JP)
Inventor: Kamoda, Hironobu
c/o Shiseido Company Ltd, 7-5-5 Ginza, Chuo-Ku
Tokyo (JP)
Inventor: Chiba, Tadahiro
Shiseido Laboratories 1050 Nippa-Cho, Kohoku-Ku
Yokohama-Shi, Kanagawa (JP)

(74) Representative: Hirsch, Marc-Roger
Cabinet Hirsch 34 rue de Bassano
F-75008 Paris (FR)

EP 0 222 670 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

BACKGROUND OF THE INVENTION

The present invention relates to a [benzo-1,2,4-thiadiazine]-1,1-oxide dissolved composition and a hair germination and hair growth promoting agent containing the same as an effective ingredient. More specifically, it relates to a dissolved composition comprising (i) one or more of [benzo-1,2,4-thiadiazine]-1,1-oxide derivatives (hereinafter called "the dioxide") and (ii) benzyl alcohol (hereinafter called "BA"), and (iii) optionally isopropyl alcohol (hereinafter called "IPA") and/or water, or (i) one or more of the dioxides, (ii) one or more of humectants (iii) IPA, (iv) water, and, optionally BA. Furthermore, it concerns a hair germination, hair growth promoting agent containing the dissolved composition, as an effective ingredient, which can be utilized in the field of pharmaceuticals or the field of cosmetics.

Dioxide has been used in the prior art as a therapeutical agent for hypertension, for injection, as a therapeutical agent for hypoglycemia due to excessive secretion of insulin, for oral administration, but the generation of hypertrichosis has been reported as a side effect thereof (see : The Journal of Pediatrics, Vol. 71, No. 4, pp. 494-505, 1967).

On the basis of this finding, an invention using dioxide as the hair germination, hair growth promoting agent by external application is disclosed in Japanese Unexamined Patent Publication (Kokai) No. 56-65811.

Dioxide, however, has a high melting point and there is no adequate solvent therefor, and thus it is mainly used in preparation forms such as tablets, granules, suspensions. If the pH is made 11.6 or higher, a certain solubility in water may be exhibited, but this is not satisfactory.

In the invention disclosed in Japanese Unexamined Patent Publication (Kokai) No. 56-65811, dimethyl sulfoxide (hereinafter called DMSO) has been used as the solvent for dioxide, and further, to alleviate the irritative property, a solvent mixture of DMSO with e.g. IPA or ethyl alcohol, is used. Water, which is the safest solvent, can be used only when used in combination with DMSO.

DMSO will, however, cause primary irritation such as erythema, etc., or contact urticaria by topical application, as reported in Contact Dermatitis Vol. 4, pp. 80-, 1978 and Archives Dermatology (Arch. Derm.), Vol. 90, pp, 512-, 1964. Accordingly, the compositions using DMSO are not preferable from a skin safety aspect or with respect to useability.

For the dissolution of dioxide, n-decylmethyl sulfoxide has been also used as the solvent, but this substance also is not preferable from the skin safety aspect, and thus there is a need to develop a preparation having an excellent skin safety and employing dioxide.

SUMMARY OF THE INVENTION

Accordingly, the objects of the present invention are to eliminate the above-mentioned disadvantages of the prior art and to provide a dissolved composition comprising dioxide, which composition has an excellent skin safety and useability, and consequently, found that BA does not cause skin irritation and exhibits an excellent dissolving characteristic similar to DMSO, and also has an excellent useability and stability.

Another object of the present invention is to provide a hair germination, hair growth promoting agent further successfully developed, which has an extremely excellent skin safety, and also has an excellent useability and stability.

Other objects and advantages of the present invention will be apparent from the following description.

In accordance with the present invention, there is provided a dissolved composition comprising (i) at least one [benzo-1,2,4-thiadiazine.]-1,1-dioxide derivative and (ii) benzyl alcohol the [benzo-1,2,4-thiadiazine]-1 1-dioxide derivative having the general formula :

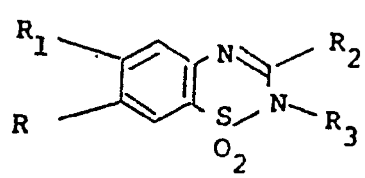

wherein R is Cl, $CF_3$, $SO_2$, or $NH_2$, $R_1$ is H, Cl, $SO_2$, or $NH_2$, $R_2$ is H, $C_nH_{2n+1}$ wherein n is an integer of 1 to 10, $CH_2OH$, COOH, or $CH_2C_6H_5$, $R_3$ is H, $C_nH_{2n+1}$ wherein n is an integer of 1 to 10 or $CH_2C_6H_5$ and wherein the amount of the [benzo-1,2,4-thiadiazine]-1,1- derivative is 0.001% to 10% by weight and the amount of

benzyl alcohol is 1.0% to 30% by weight, both based on the total weight of the composition.

In accordance with the present invention, there is also provided a dissolved composition comprising (i) at least one [benzo-1,2,4-thiadiazine]-1,1-dioxide derivative, (ii) BA, and (iii) IPA and/or water, wherein the pH of the system when containing water is 8.5 to 11.0.

In accordance with the present invention, there is further provided a dissolved composition comprising (i) at least one [benzo-1,2,4-thiadiazine]-1,1-dioxide derivative, (ii) at least one humectant, (iii) IPA, (iv) water, and (v) optionally BA, wherein the pH of the system is 8.5 to 11.0.

In accordance with the present invention, there is further provided a hair germination and hair growth promoting agent containing, as an effective ingredient, the above-mentioned dissolved composition.

DESCRIPTION OF THE PREFERRED EMBODIMENTS

The dioxide to be used in the present invention is a substance known as a therapeutical agent for hypertension and as a therapeutical agent for hypoglycemia based on an excessive secretion of insulin, and is a compound represented by the formula shown below

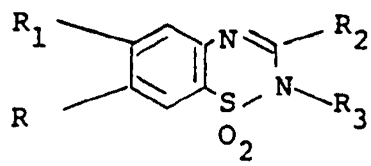

wherein R is Cl, $CF_3$, $SO_2$, or $NH_2$, $R_1$ is H, Cl, $SO_2$, or $NH_2$, $R_2$ is H, $C_nH_{2n+1}$ wherein n is an integer of 1 to 10, $CH_2OH$, COOH, or $CH_2C_6H_5$, $R_3$ is H, $C_nH_{2n+1}$ wherein n is an integer of 1 to 10, or $CH_2C_6H_5$.

The amount of the dioxide formulated may be 0.001 to 10% by weight, preferably 0.005 to 10% by weight in the dissolved composition of the present invention. When used as the hair germination, hair growth promoting agent, the hair germination, hair growth promoting effect becomes greater as the formulated amount is increased, but in view of the revelation of the occurrence of side effects when a large amount is used, it may be less than 10%. Preferably, the amount used is 0.01% to 7% by weight.

The amount of BA used as the solvent may be 1.0% to 30% by weight, preferably 3.0% to 15% by weight. Further, the amount of IPA formulated may be 10% to 85% by weight, preferably 20% to 60% by weight. The amount of water when formulated may be 10% to 70%, preferably 20% to 50% by weight. The amounts formulated may be suitably selected in accordance with the amount of dioxide formulated and the combination of the solvents.

As the pH controller in the above-mentioned system when water is formulated, either an inorganic base or an organic base may be used, provided that the pH is controlled to 8.5 to 11.0, preferably 9.5 to 11.0. In the case of a pH lower than 8.5, the solubility of the dioxide is not satisfactory. On the other hand, a pH of over 11.0 is not desirable from safety aspect. Particularly, when the dissolved composition of the dioxide is controlled, pH control is important, and thus it is desirable to use a buffer solution. When a 3% by weight, dissolved solution of 7-chloro-3-methyl-2H-[benzo-1,2,4-thiadiazine]-1,1-dioxide is to be prepared, if 10% by weight of BA is used and the pH is controlled to 10 by using a disodium phosphate - sodium hydroxide buffer, the mixing ratio of IPA to water may be appropriately 8 : 1 to 1 : 2. When it is desired to control the pH to a lower value, an increased amount of BA may be added.

The humectants, usable in the second embodiment of the present invention include, for example, polyethylene glycols, propylene glycol, dipropylene glycol, glycerin, 1,3-butylene glycol, ethylene glycol, diethylene glycol, sorbitol, maltitol, hyaluronic acid, sodium lactate, bile acid salt, dl-pyrrolidone carboxylic acid salt, preferably polyethylene glycols, dipropylene glycol, sodium lactate and dl-pyrrolidone acid salts. These humectants can be formulated alone or in any mixture thereof, and the total amount of the humectant formulated may be 1.0% to 50% by weight, preferably 3.0% to 40% by weight. The amount of IPA formulated may be 10% to 85% by weight, preferably 20% to 60% by weight. The amount of water formulated may be 1.0% to 70% by weight, preferably 3.0 to 50% by weight. These amounts formulated may be suitably selected in accordance with the amount of dioxide formulated and the combination of the solvents.

As the pH controller, either inorganic bases or organic bases, or a buffering agent utilizing these salts may be used, provided that the pH is controlled to 8.5 to 11.0. In the case of a pH lower than 8.5, the solubility of the dioxide is not satisfactory. On the other hand, a pH of over 11.0 is not desirable from a safety aspect. For example, when a 3% dissolved solution of 7-chloro-3-methyl-2H-[benzo-1,2,4-thiadiazine]-1,1-dioxide is to be prepared, if 40% of IPA is used and the pH is controlled to 10 by using an aqueous sodium hydroxide solution, the mixing ratio of a humectant to water may be appropriately 10 : 1 to 1 : 2.

The hair germination and hair growth promoting agent according to the present invention can be further formulated by, in addition to the dioxide, sterilizers such as salicylic acid, resorcin and hexachlorophen ; vitamins such as nicotinic acid, vitamin E, vitamin A acid, pantothenic acid, ethynyl estradiol, hinokitiol, glycyrrhetic acid and biotin ; and chemicals such as fatty acids and amino acids, which are generally used in hair germination, hair promoting agents. Also, if desired, and if within the scope which will not impair the effect of the present invention, it is possible to formulate various components generally employed for pharmaceuticals or cosmetics, namely aqueous components, powder components, oil components, surfactants, humectants, thickeners, preservatives, antioxidants, perfumes and colorants.

The dissolved composition of the present invention contains a dioxide having an excellent skin safety, useability, and is an excellent preparation having a good stability with a lapse of time. Further, it can be made into a transparent gel-like dissolved composition by the addition of a thickener.

The solubilities of 7-chloro-3-methyl-2H[benzo-1,2,4-thiadiazine]-1,1-dioxide in various solvents at room temperature are shown in Table 1.

Table 1

| Solvent | Dioxide*1 (% by weight) | | | |
|---|---|---|---|---|
| | 0.2 | 1.0 | 2.0 | 3.0 |
| DMSO | o | o | o | o |
| BA | o | o | x | x |
| IPA | o | x | x | x |
| Ethyl alcohol | x | x | x | x |
| DPG*2 | x | x | x | x |
| BA/IPA = 1/9 | o | o | x | x |
| IPA/DPG = 1/1 | o | x | x | x |
| BA/IPA/aqueous alkali solution (pH = 10) = 1/3/6 | o | o | o | o |
| IPA/DPG/aqueous alkali solution (pH = 10) = 2/1/1 | o | o | o | o |

*1 : The dioxide is 7-chloro-3-methyl-2H-[benzo-1,2,4-thiadiazine]-1,1-dioxide

*2 : Dipropylene glycol

In the Table, o : completely and transparently dissolved ;
x : partially not dissolved, with presence of crystals.

As apparent from Table 1, by using BA, or by using IPA, humectant, and water under the specified pH, a transparent dissolved composition can be obtained without using DMSO at all.

The skin safety of the solvent system to be used in the present invention is shown below, compared with that of the solvent system of the prior art.

[Test Method]

By the use of 6 white rabbits, a primary skin irritation test was conducted according to the Draize method. That is, 0.5 ml of each sample was applied to a hair-removed site of the backs of the rabbits, and the presence of erythma and edema was determined 24 hours and 72 hours later. The average values of the respective ratings are shown in Table 2.

Standards for rating:

0 - 2: weak irritation

3 - 5: moderate irritation

6 - 8: strong irritation

Table 2

| Sample | Irritation rating |
|---|---|
| DMSO/ethyl alcohol = 1/3 | 5 |
| DMSO/IPA = 1/9 | 5 |
| DMSO/water = 1/1 | 3 |
| BA/IPA/aqueous alkali solution (pH = 10) = 1/3/6 | 0 |
| IPA/DPG/aqueous alkali solution (pH = 10) = 2/1/1 | 0 |
| BA/IPA = 1/9 | 0 |

As apparent from Table 2, the solvent system according to the present invention has an extremely high safety factor, compared with the solvent system of the prior art.

EXAMPLES

The present invention will now be further illustrated by, but is by no means limited to, the following Examples, wherein "%" is on a weight basis unless otherwise specified.

Example 1 Transparent Liquid Composition

| | | |
|---|---|---|
| (1) | 7-chloro-3-methyl-2H-[benzo-1,2,4-thiadiazine]-1,1-dioxide | 0.05% |
| (2) | BA | 10.0 |
| (3) | 1,3-butylene glycol | 5.0 |
| (4) | IPA | 84.9 |
| (5) | vitamin E | 0.05 |

Preparation method

(1) was added to (2), and the mixture was heated to 50°C to dissolve (1) under stirring. Then, (4), (3) and (5) were successively added and dissolved under stirring to give a transparent liquid composition.

Example 2 Transparent Liquid Composition

| | | |
|---|---|---|
| (1) | 7-chloro-3-methyl-2H-[benzo-1,2,4-thiadiazine]-1,1-dioxide | 10.0% |
| (2) | BA | 30.0 |
| (3) | IPA | 15.0 |
| (4) | 1,3-butylene glycol | 4.0 |
| (5) | glycerine | 2.0 |
| (6) | potassium hydroxide | 2.52 |
| (7) | deionized water | 36.48 |

Preparation method

(1) was added to (2), and the mixture was heated to 50 - 55°C, stirring was then effected, and thereafter (3), (4) and (5) were successively added. (7) was added to (6), and the solution obtained by stirring was added gradually to the solvent phase previously prepared under stirring, to give a transparent liquid composition having a pH of about 10.2.

This composition is applicable for hair growth shampoos, etc.

Example 3 Transparent Liquid Composition

| | | |
|---|---|---|
| (1) | 7-chloro-3-methyl-2H-[benzo-1,2,4-thiadiazine]-1,1-dioxide | 7.0% |
| (2) | BA | 25.0 |

| | | |
|---|---|---|
| (3) | IPA | 25.0 |
| (4) | propylene glycol | 5.0 |
| (5) | glycerin | 2.0 |
| (6) | sodium hydroxide | 1.05 |
| (7) | trisodium phosphate | 0.15 |
| (8) | deionized water | 34.8 |

Preparation method

Similar to Example 2.

Example 4 Transparent Liquid Composition

| | | |
|---|---|---|
| (1) | 7-chloro-3-methyl-2H-[benzo-1,2,4-thiadiazine]-1,1-dioxide | 3.0% |
| (2) | BA | 10.0 |
| (3) | IPA | 50.0 |
| (4) | glycerin | 4.0 |
| (5) | sodium hydroxide | 0.4 |
| (6) | deionized water | 32.6 |

Preparation method

Similar to Example 2.

To a patient (male, age 35) bald over a considerably broad range at the parietal and occipital region, this preparation was applied to the scalp at the bald portion at a dose of 2 - 4 ml twice per day. After 4 months, at the applied area, a dense growth of vellus hair was observed, and thereafter, it was confirmed that a part thereof gradually thickened to become the terminal hair.

Example 5 Transparent Liquid Composition

| | | |
|---|---|---|
| (1) | 7-chloro-3-methyl-2H-[benzo-1,2,4-thiadiazine]-1,1-dioxide | 1.0% |
| (2) | BA | 15.0 |
| (3) | 1,3-butylene glycol | 25.0 |
| (4) | glycerin | 10.0 |
| (5) | hydrogeneted castor oil derivative (POE: 60 mole) | 5.0 |
| (6) | sodium hydroxide | 0.17 |
| (7) | deionized water | to 100.0 |

Preparation method

(1) was added to (2), and heated to 50°C and dissolved therein under stirring. Further, (7) was added to be dissolved, and (3) and (4) were added, followed by stirring well. Then, a solution of (6) dissolved in (7) was added to give a transparent liquid preparation having a pH of about 9.8. To a 30 years old male

with alopecia at the parietal region, this preparation was applied to the scalp at a dose of 2 - 4 ml twice per day. After 3 months, at the applied area, a growth of vellus hair was observed, and thereafter, it was found to gradually thicken and become the terminal hair.

Example 6 Transparent Liquid Composition

| | | |
|---|---|---|
| (1) | 7-chloro-3-methyl-2H-[benzo-1,2,4-thiadiazine]-1,1-dioxide | 0.05% |
| (2) | BA | 10.0 |
| (3) | propylene glycol | 5.0 |
| (4) | 1,3-butylene glycol | 10.0 |
| (5) | glycerin | 10.0 |
| (6) | ethyl alcohol | 69.0 |
| (7) | POE (15 mole) oleyl alcohol ether | 4.0 |

Preparation method

(1) was added to (2), and heated to 50°C and dissolved therein under stirring. Further, (6) and (7) were added and, under stirring, (4), (5), and (6) were added to give a transparent liquid preparation. To a 32 years old male with baldness at the parietal region, this preparation was applied to the scalp at the parietal region and the whole head at a dose of 4 to 6 ml twice per day. After 1 month, hair loss was dramatically reduced, and hair at the bald parietal region thickened after about 3 months, and gradually became the terminal hair.

Example 6 Gel-like Hair Growth Composition

| | | |
|---|---|---|
| (1) | 7-chloro-3-methyl-2H-[benzo-1,2,4-thiadiazine]-1,1-dioxide | 2.0% |
| (2) | vitamin E acetate | 0.05 |
| (3) | hinokitiol | 0.1 |
| (4) | pantothenyl ethyl ether | 0.05 |
| (5) | BA | 10.0 |
| (6) | glycerin | 5.0 |
| (7) | hydrogenated castor oil derivative | 4.0 |
| (8) | IPA | 50.0 |
| (9) | hydroxypropylcellulose-H | 1.0 |
| (10) | carboxyvinyl polymer | 0.5 |
| (11) | sodium hydroxide | 0.6 |
| (12) | deionized water | 25.7 |

Preparation method

(1) was added to (5), and the mixture heated to 50°C and then thoroughly stirred. Next, (8), (9) were successively added, followed by stirring. Further, an alkali aqueous solution containing (11) dissolved in (12) was gradually added to completely dissolve (1), and thereafter, a solution of (2), (3), (4) and (7) dissolved in a part of (8), and (6) was gradually added and dissolved under stirring. Finally, a solution of (10) previously dissolved in a part of (12) was added and thoroughly stirred to give a transparent gel-like composition.

Example 7 Gel Type Hair Growth Preparation

| | | |
|---|---|---|
| (1) | 7-chloro-3-methyl-2H-[benzo-1,2,4-thiadiazine]-1,1-dioxide | 0.01% |
| (2) | BA | 5.0 |
| (3) | IPA | 18.0 |
| (4) | ethyl alcohol | 20.0 |
| (5) | 1,3-butylene glycol | 10.0 |
| (6) | ethynyl estradiol | 0.002 |
| (7) | hydroxypropyl cellulose | 0.8 |
| (8) | carboxyvinyl polymer | 0.5 |
| (9) | diisopropanolamine | 1.6 |
| (10) | deionized water | 44.088 |

Preparation method

Similar to Example 3

Example 8 Hair Tonic

| | | |
|---|---|---|
| (1) | 7-chloro-3-methyl-2H-[benzo-1,2,4-thiadiazine]-1,1-dioxide | 0.05% |
| (2) | hinokitiol | 0.01 |
| (3) | vitamin $B_6$ | 0.1 |
| (4) | pantothenyl ethyl ether | 0.1 |
| (5) | vitamin E | 0.01 |
| (6) | BA | 10.0 |
| (7) | propylene glycol | 5.0 |
| (8) | 1,3-butylene glycol | 10.0 |
| (9) | glycerine | 2.0 |
| (10) | ethyl alcohol | 68.13 |
| (11) | perfume | 0.6 |
| (12) | POE (15 mole) oleyl alcohol ether | 4.0 |

Preparation method

(1) was added to (6), heated to 50°C and dissolved therein under stirring. Next, (10) and (12) were added, and (2), (3), (4), (5) and (11) were successively added under stirring. Further, (7), (8) and (9) were added to give a hair tonic of a transparent liquid preparation.

Example 9 Transparent Liquid Composition

| | | |
|---|---|---|
| (1) | 7-chloro-3-methyl-2H-[benzo-1,2,4-thiadiazine]-1,1-dioxide | 0.01% |
| (2) | dipropylene glycol | 20.0 |
| (3) | 1,3-butylene glycol | 5.0 |
| (4) | IPA | 60.0 |
| (5) | potassium hydroxide | 0.015 |
| (6) | purified water | balance |

Preparation method

After (5) was added to (6) to be dissolved therein, (1) was added and the mixture heated to 50°C to dissolve (1). Next, (2), (3), and (4) were successively added, followed by mixing with stirring, to give a transparent liquid composition. The transparent liquid composition had a pH of about 8.5.

Example 10 Transparent Liquid Composition

| | | |
|---|---|---|
| (1) | 7-chloro-3-methyl-2H-[benzo-1,2,4-thiadiazine]-1,1-dioxide | 10.0% |
| (2) | polyethylene glycol 200 | 5.0 |
| (3) | aqueous sodium pyrrolidone-carboxylate solution (50%) | 30.0 |
| (4) | IPA | 50.0 |
| (5) | sodium hydroxide | 1.6 |
| (6) | purified water | balance |

Preparation method

After adding and dissolving (5) in (6), (1) was added and heated to 50°C to well disperse (1) in the solution. Next, (2), (3), and (4) were successively added, followed by mixing with stirring to dissolve (1), whereby a transparent liquid composition was obtained. The transparent liquid composition had a pH of about 10.7.

Example 11 Transparent Liquid Composition

| | | |
|---|---|---|
| (1) | 7-chloro-3-methyl-2H-[benzo-1,2,4-thiadiazine]-1,1-dioxide | 6.0% |
| (2) | polyethylene glycol 300 | 15.0 |
| (3) | aqueous sodium lactate solution (50%) | 15.0 |
| (4) | IPA | 40.0 |
| (5) | sodium hydroxide | 1.05 |
| (6) | trisodium citrate | 0.05 |
| (7) | purified water | balance |

Preparation method

(5), (6) were added to (7), and thereafter, the procedure of Example 10 was followed. The transparent liquid composition had a pH of about 10.3.

Example 12 Transparent Liquid Composition

| | | |
|---|---|---|
| (1) | 7-chloro-3-methyl-2H-[benzo-1,2,4-thiadiazine]-1,1-dioxide | 1.0% |
| (2) | dipropylene glycol | 25.0 |
| (3) | aqueous sodium pyrrolidone-carboxylate solution (50%) | 10.0 |
| (4) | IPA | 30.0 |
| (5) | potassium hydroxide | 0.23 |
| (6) | purified water | balance |

Preparation method

Similar to Example 10. The transparent liquid composition had a pH of about 9.0.

Effect

To a patient (male, age 33) bald over a considerably broad range at the parietal and occipital regions, this preparation was applied to the scalp at the bald area at a dose of 2 - 4 ml twice per day. After 4 months, at the applied area, a dense growth of vellus hair was observed, and thereafter, it was confirmed that a part thereof gradually hardened to become the terminal hair.

Example 13 Transparent Liquid Composition

| | | |
|---|---|---|
| (1) | 7-chloro-3-methyl-2H-[benzo-1,2,4-thiadiazine]-1,1-dioxide | 3.0% |
| (2) | 1,3-butylene glycol | 10.0 |
| (3) | aqueous sodium lactate solution (50%) | 10.0 |
| (4) | IPA | 50.0 |
| (5) | polyoxyethylene hydrogenated castoroil (POE: 60 mole) | 2.0 |
| (6) | sodium hydroxide | 0.5 |
| (7) | purified water | balance |

Preparation method

(5), (6) were added to (7), and the mixture heated to 50 - 55°C to dissolve (5), (6), and thereafter, the procedure of Example 10 was followed. The transparent liquid composition had a pH of about 10.2.

Effect

To a patient (male, age 35) bald at the parietal region, this preparation was applied to the scalp at a dose of 2 - 4 ml twice per day. After about 2 months, at the applied area, a growth of vellus hair was observed, and thereafter it was found to have gradually thickened until becoming the terminal hair, about one year later.

Example 14 Transparent Liquid Composition

| | | |
|---|---|---|
| (1) | 7-chloro-3-methyl-2H-[benzo-1,2,4-thiadiazine]-1,1-dioxide | 5.0% |
| (2) | dipropylene glycol | 15.0 |
| (3) | aqueous sodium lactate solution (50%) | 10.0 |
| (4) | IPA | 40.0 |
| (5) | BA | 10.0 |
| (6) | sodium hydroxide | 0.9 |
| (7) | purified water | balance |

Preparation method

Similar to Example 10. The transparent liquid composition had a pH of about 10.6.

Example 15 Hair Tonic:

| | | |
|---|---|---|
| (1) | 7-chloro-3-methyl-2H-[benzo-1,2,4-thiadiazine]-1,1-dioxide | 0.03% |
| (2) | hinokitiol | 0.01 |
| (3) | vitamin E acetate | 0.05 |
| (4) | vitamin $B_6$ | 0.1 |
| (5) | propylene glycol | 5.0 |
| (6) | polyethylene glycol 400 | 10.0 |
| (7) | IPA | 25.0 |
| (8) | ethyl alcohol | 45.0 |
| (9) | perfume | q.s. |
| (10) | polyoxyethylene (15 mole) oleyl alcohol | 4.0 |
| (11) | sodium hydroxide | 0.05 |
| (12) | purified water | balance |

Preparation method

After (11) was added and dissolved in (12), (1) was added to the solution, and the mixture heated to 50°C. To this mixture were added successively (5), (6), and (7), followed by mixing with stirring, to dissolve (1) and give a transparent liquid composition. This was called composition (A).

Separately, (2), (3), (4), (9), (10) were successively added and dissolved in (8) under stirring. This was called composition (B). Which stirring composition (A), composition (B) was gradually added, followed by mixing with stirring, and the resultant mixture was filtered to give a hair tonic of a transparent liquid preparation.

Example 16 Gel-like Hair Growth Preparation

| | | |
|---|---|---|
| (1) | 7-chloro-3-methyl-2H-[benzo-1,2,4-thiadiazine]-1,1-dioxide | 2.0% |
| (2) | hinokitiol | 0.01 |
| (3) | pantothenyl ethyl ether | 0.05 |
| (4) | dipropylene glycol | 15.0 |
| (5) | glycerin | 5.0 |
| (6) | IPA | 50.0 |
| (7) | hydroxypropyl cellulose | 1.0 |
| (8) | carboxyvinyl polymer | 1.0 |
| (9) | polyoxyethylene hydrogenated castoroil (P.O.E.: 60 mole) | 2.0 |
| (10) | sodium hydroxide | 0.3 |
| (11) | purified water | balance |

Preparation method

After (10) was added and dissolved in a part of (11), (1) was added to the solution, the mixture heated to 50°C, and (1) was well dispersed under stirring. To the dispersion were added (4), (5), and a part of (6), and the mixture stirred to effect complete dissolution and give a transparent liquid composition (A).

Separately, (2), (3), (9) were dissolved in a part of (6) and (7) was dispersed in the solution to prepare a composition (B). Further, (8) is dispersed in a part of (11) to prepare a composition (C).

While stirring composition (B), composition (C) was added thereto, followed by mixing well. Further, to the mixture was gradually added composition (A), followed by mixing with stirring, to give a transparent gel-like composition.

Example 17 Gel-like Hair Growth Agent

| | | |
|---|---|---|
| (1) | 7-chloro-3-methyl-2H-[benzo-1,2,4-thiadiazine]-1,1-dioxide | 0.02% |
| (2) | ethynyl estradiol | 0.002 |
| (3) | vitamin E acetate | 0.05 |
| (4) | 1,3-butadiene glycol | 5.0 |
| (5) | diethylene glycol | 10.0 |
| (6) | glycerin | 4.0 |
| (7) | IPA | 20.0 |
| (8) | ethyl alcohol | 25.0 |
| (9) | hydroxypropyl cellulose | 1.2 |
| (10) | carboxyvinyl polymer | 0.8 |
| (11) | diisopropanolamine | 0.3 |
| (12) | potassium hydroxide | 0.02 |
| (11) | purified water | balance |

Preparation method

Similar to Example 16, but the transparent liquid composition (A) was constituted by (1), (4), (5), (6), (7), (11), (12), and a part of (13), the composition (B) by (2), (3), (8), (9), and the composition (C) by (10) and a part of (13).

Example 18 Emulsion

| | | |
|---|---|---|
| (1) | 7-chloro-3-methyl-2H-[benzo-1,2,4-thiadiazine]-1,1-dioxide | 0.05% |
| (2) | dipropylene glycol | 15.0 |
| (3) | IPA | 20.0 |
| (4) | liquid paraffin | 3.0 |
| (5) | cetyl alcohol | 0.2 |
| (6) | carboxyvinyl polymer | 0.2 |
| (7) | perfume | q.s. |
| (8) | polyoxyethylene hydrogenated castor oil (P.O.E.: 40 mole) | 1.0 |
| (9) | preservative | q.s. |
| (10) | sodium hexametaphosphate | 0.03 |
| (11) | potassium hydroxide | 0.17 |
| (12) | purified water | balance |

Preparation method

After (11) was added and dissolved in a part of (12), (1) was added to the solution, the mixture heated to 50°C, and a part of (2) and (3) were added to be dissolved therein. This was called composition (A).

To the remainder of (2) were added and dissolved a part of (12) and (8) while heating at 50°C. While the solution was stirred by a homomixer, a mixture prepared by adding (5), (7), (8), (9) to (4) under heating at 70°C was gradually added to be emulsified therein. This was called composition (B).

After (6) and (10) were added and dissolved in the remainder of (12), while stirring of the solution, composition (B) and composition (A) were successively added thereto. Further, the mixture was mixed while stirring by a mixer and then cooled to obtain an emulsion.

Example 19 Cream:

| | | |
|---|---|---|
| (1) | 7-chloro-3-methyl-2H-[benzo-1,2,4-thiadiazine]-1,1-dioxide | 0.1% |
| (2) | vitamin E acetate | 0.05 |
| (3) | polyethylene glycol 200 | 13.0 |
| (4) | aqueous sodium lactate solution (50%) | 5.0 |
| (5) | IPA | 25.0 |

| | | |
|---|---|---|
| (6) | liquid paraffin | 1.0 |
| (7) | castor oil | 3.5 |
| (8) | perfume | q.s. |
| (9) | glycerine mono-fatty acid ester | 1.5 |
| (10) | preservative | q.s. |
| (11) | clay mineral (bentonite) | 6.0 |
| (12) | potassium hydroxide | 0.03 |
| (13) | purified water | balance |

Preparation method

After (12) was added and dissolved in a part of (13), (1) was added and the mixture heated to 50°C. Then, (3), (4), (5) were added to dissolve (1). This was called composition (A).

To (6) were successively added (2), (7), (8), (9), (10), the mixture heated to 70°C, and mixed to prepare a solution. This was called composition (B).

At a temperature maintained at 70°C, while stirring composition (A), composition (B) was gradually added to effect preliminary emulsification, followed by emulsification by a homomixer.

This was added to a dispersion previously prepared by adding (11) to the remainder of (13) under stirring, and the mixture then cooled to give a cream.

Example 20 Aerosol:

Stock solution recipe

| | | |
|---|---|---|
| (1) | 7-chloro-3-methyl-2H-[benzo-1,2,4-thiadiazine]-1,1-dioxide | 2.0% |
| (2) | ethynyl estradiol | 0.001 |
| (3) | pantothenyl ethyl ether | 0.05 |
| (4) | dipropylene glycol | 15.0 |
| (5) | IPA | 40.0 |
| (6) | ethyl alcohol | 37.0 |
| (7) | polyoxyethylene hydrogenated castor oil (P.O.E.: 60 mole) | 1.0 |
| (8) | perfume | q.s. |
| (9) | sodium hydroxide | 0.35 |
| (10) | purified water | balance |

Filling recipe

| | | |
|---|---|---|
| (11) | Stock solution | 30.0% |
| (12) | Freon 12 | 42.0 |
| (13) | Freon 13 | 28.0 |

Preparation method

The stock solution was prepared in the same way as in Example 15.

Filling was carried out by filling the stock solution (11) at a prescribed amount into a can and, after mounting a valve, the gases (12) and (13) were successively filled in prescribed amounts.

**Claims**

1. A dissolved composition comprising (i) at least one [benzo-1,2,4-thiadiazine]-1,1-dioxide derivative and (ii) benzyl alcohol, the [benzo-1,2,4-thiadiazine]-1,1-dioxide derivative having the general formula :

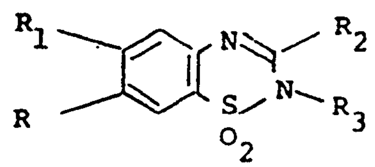

wherein R is Cl, $CF_3$, $SO_2$, or $NH_2$, $R_1$ is H, Cl, $SO_2$, or $NH_2$, $R_2$ is H, $C_nH_{2n+1}$ wherein n is an integer of 1 to 10, $CH_2OH$, COOH, or $CH_2C_6H_5$, $R_3$ is H, $C_nH_{2+1}$ wherein n is an integer of 1 to 10 or $CH_2C_6H_5$ and wherein the amount of the [benzo-1,2,4-thiadiazine]-1,1- derivative is 0.001% to 10% by weight and the amount of benzyl alcohol is 1.0% to 30% by weight, both based on the total weight of the composition.

2. A dissolved composition comprising (i) at least one [benzo-1,2,4-thiadiazine]-1,1- dioxide derivative, (ii) benzyl alcohol and (iii) isopropyl alcohol, water, or the mixture thereof, the pH of the system when containing water being 8.5 to 11.0, the [benzo-1,2,4-thiadiazine]-1,1- dioxide derivative having the general formula ;

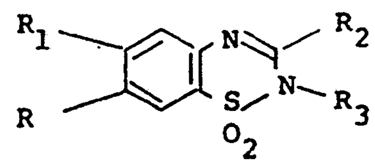

wherein R is Cl, $CF_3$, $SO_2$, or $NH_2$, $R_1$ is H, Cl, $SO_2$, or $NH_2$, $R_2$ is H, $C_nH_{2n+1}$ wherein n is an integer of 1 to 10, $CH_2OH$, COOH, or $CH_2C_6H_5$,$R_3$ is H, $C_nH_{2n+1}$ wherein n is an integer of 1 to 10 or $CH_2C_6H_5$ and wherein the amount of the [benzo-1,2,4-thiadiazine]-1,1- derivative is 0.001% to 10% by weight and the amount of benzyl alcohol is 1.0% to 30% by weight, the amount of isopropyl alcohol is 10% to 85% by weight, the amount of water is 10% to 70% by weight, all based on the total weight of the composition.

3. A dissolved composition comprising (i) at least one [benzo-1,2,4-thiadiazine]-1,1- dioxide derivative, (ii) at least one humectant, (iii) isopropyl alcohol, and (iv) water, the pH of the system being 8.5 to 11.0, the [benzo-1,2,4-thiadiazine]-1,1- dioxide derivative having the general formula :

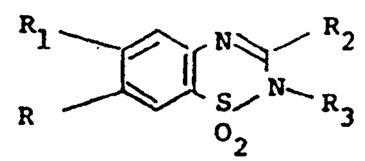

wherein R is Cl, $CF_3$, $SO_2$, or $NH_2$, $R_1$ is H, Cl, $SO_2$, or $NH_2$, $R_2$ is H, $C_nH_{2n+1}$ wherein n is an integer of 1 to 10, $CH_2OH$, COOH, or $CH_2C_6H_5$, $R_3$ is H, $C_nH_{2n+1}$ wherein n is an integer of 1 to 10 or $CH_2C_6H_5$ and wherein the amount of the [benzo-1,2,4-thiadiazine]-1,1- derivative is 0.001% to 10% by weight, the amount of the humectant is 1.0% to 50% by weight, the amount of isopropyl alcohol is 10% to 85% by weight, and the amount of water is 10% to 70% by weight, all based on the total weight of the composition.

4. A dissolved composition comprising (i) at least one [benzo-1,2,4-thiadiazine]-1,1- dioxide derivative, (ii) at least one humectant, (iii) benzyl alcohol, (iv), isopropyl alcohol, and (v) water, the pH of the system being 8.5 to 11.0. the [benzo-1,2,4-thiadiazine]-1,1- dioxide derivative having the general formula :

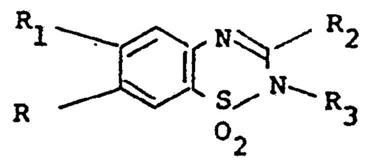

wherein R is Cl, $CF_3$, $SO_2$, or $NH_2$, $R_1$ is H, Cl, $SO_2$, or $NH_2$, $R_2$ is H, $C_nH_{2n+1}$ wherein n is an integer of 1 to 10, $CH_2OH$, COOH, or $CH_2C_6H_5$, $R_3$ is H, $C_nH_{2n+1}$ wherein n is an integer of 1 to 10 or $CH_2C_6H_5$ and wherein the amount of the [benzo-1,2,4-thiadiazine]-1,1- derivative is 0.001% to 10% by weight, the amount of the humectant is 1.0% to 50% by weight, the amount of benzyl alcohol is 1.0% to 30% by weight, the amount of isopropyl alcohol is 10% to 85% by weight, and the amount of water is 10% to 70% by weight, all based on the total weight of the composition.

5. A hair germination and hair growth promoting agent containing as an effective ingredient a dissolved composition comprising (1) at least one [benzo-1,2,4-thiadiazine]-1,1dioxide derivative, and (ii) benzyl alcohol, the [benzo-1,2,4-thiadiazine]-1,1- dioxide derivative having the general formula :

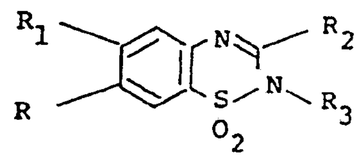

wherein R is Cl, $CF_3$, $SO_2$, or $NH_2$, $R_1$ is H, Cl, $SO_2$, or $NH_2$, $R_2$ is H, $C_nH_{2n+1}$ wherein n is an integer of 1 to 10, $CH_2OH$, COOH, or $CH_2C_6H_5$, $R_3$ is H, $C_nH_{2n+1}$ wherein n is an integer of 1 to 10 or $CH_2C_6H_5$ and wherein the amount of the [benzo-1,2,4-thiadiazine]-1,1- derivative is 0.001% to 10% by weight and the amount of the benzyl alcohol is 1.0% to 30% by weight, both based on the total weight of the composition.

6. A hair germination and hair growth promoting agent containing as an effective ingredient a dissolved composition comprising (i) at least one [benzo-1,2,4-thiadiazine]-1,1dioxide derivative, (ii) benzyl alcohol, and (iii) isopropyl alcohol, water, or the mixture thereof, the pH of the system when containing water being 8.5 to 11.0, the [benzo-1,2,4-thiadiazine]-1,1- dioxide derivative having the general formula :

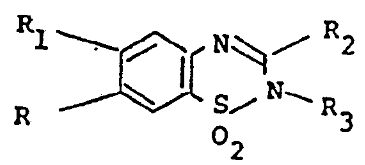

wherein R is Cl, $CF_3$, $SO_2$, or $NH_2$, $R_1$ is H, Cl, $SO_2$, or $NH_2$, $R_2$ is H, $C_nH_{2+1}$ wherein n is an integer of 1 to 10, $CH_2OH$, COOH, or $CH_2C_6H_5$, $R_3$ is H, $C_nH_{2n+1}$ wherein n is an integer of 1 to 10 or $CH_2C_6H_5$ and wherein the amount of the [benzo-1,2,4-thiadiazine]-1,1- derivative is 0.001% to 10% by weight, the amount of the benzyl alcohol is 1.0% to 30% by weight, the amount of isopropyl alcohol is 10% to 85% by weight, and the amount of water is 10% to 70% by weight, all based on the total weight of the composition.

7. A hair germination and hair growth promoting agent containing as an effective ingredient a dissolved composition comprising (i) at least one [benzo-1,2,4-thiadiazine]-1,1dioxide derivative, (ii) at least one humectant, (iii) isopropyl alcohol, and (iv) water, the pH of the system being 8.5 to 11.0, the [benzo-1,2,4-thiadiazine]-1,1- dioxide derivative having the general formula :

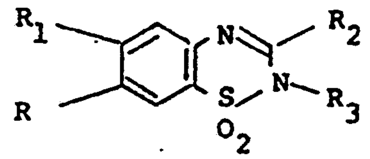

wherein R is Cl, $CF_3$, $SO_2$, or $NH_2$, $R_1$ is H, Cl, $SO_2$, or $NH_2$, $R_2$ is H, $C_nH_{2n+1}$ wherein n is an integer of 1 to

10, $CH_2OH$, COOH, or $CH_2C_6H_5$, $R_3$ is H, $C_nH_{2+1}$ wherein n is an integer of 1 to 10 or $CH_2C_6H_5$ and wherein the amount of the [benzo-1,2,4-thiadiazine]-1,1- derivative is 0.001% to 10% by weight, the amount of the humectant is 1.0% to 50% by weight, the amount of isopropyl alcohol is 10% to 85% by weight, and the amount of water is 10% to 70% by weight, all based on the total weight of the composition.

8. A hair germination and hair growth promoting agent containing as an effective ingredient a dissolved composition comprising (i) at least one [benzo-1,2,4-thiadiazine]-1,1dioxide derivative, (ii) at least one humectant, (iii) benzyl alcohol, (iv) isopropyl alcohol, and (v) water, the pH of the system being 8.5 to 11.0, the [benzo-1,2,4-thiadiazine]-1,1dioxide derivative having the general formula :

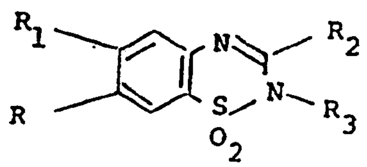

wherein R is Cl, $CF_3$, $SO_2$, or $NH_2$, $R_1$ is H, Cl, $SO_2$, or $NH_2$, $R_2$ is H, $C_nH_{2n+1}$ wherein n is an integer of 1 to 10, $CH_2OH$, COOH, or $CH_2C_6H_5$, $R_3$ is H, $C_nH_{2n+1}$ wherein n is an integer of 1 to 10 or $CH_2C_6H_5$ and wherein the amount of the [benzo-1,2,4-thiadiazine]-1,1- derivative is 0.001% to 10% by weight, the amount of the humectant is 1.0% to 50% by weight, the amount of benzyl is 1.0% to 30% by weight, the amount of isopropyl alcohol is 10% to 85% by weight, and the amount of water is 1.0% to 70% by weight, all based on the total weight of the composition.

## Ansprüche

1. Eine Zusammensetzung in Form einer Lösung, enthaltend (i) wenigstens ein (Benzo-1,2,4-thiadiazin)-1,1-dioxydDerivat und (ii) einen Benzylalkohol, wobei das (Benzo-1,2,4-thiadiazin)-1,1-dioxyd-Derivat der folgenden allgemeinen Formel entspricht :

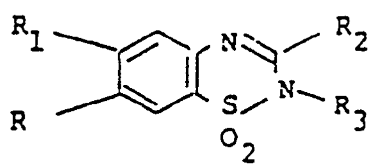

in welcher

R = Cl, $CF_3$, $SO_2$ oder $NH_2$,

$R_1$ = H, Cl, $SO_2$ oder $NH_2$,

$R_2$ = H, $C_nH_{2n+1}$ (wo n = eine ganze Zahl von 1 bis 10), $CH_2OH$, COOH oder $CH_2C_6H_5$,

$R_3$ = H, $C_nH_{2n+1}$ (wo n = eine ganze Zahl von 1 bis 10) oder $CH_2C_6H_5$,

wobei auf das Gesamtgewicht der Zusammensetzung der Anteil an (Benzo-1,2,4-thiadiazin)-1,1=Derivat 0,001 bis 10 Gew.-% und der Anteil an Benzylalkohol 1,0 bis 30 Gew.-% beträgt.

2. Eine Zusammensetzung in Form einer Lösung, enthaltend (i) wenigstens ein (Benzo-1,2,4-thiadiazin)-1,1-dioxydDerivat, (ii) Benzylalkohol und (iii) Isopropylalkohol, Wasser oder ein Gemisch derselben, wobei der pH-Wert des Systems in Gegenwart von Wasser 8,5 bis 11,0 beträgt und das (Benzo1,2,4-thiadiazin)-1,1-dioxyd-Derivat der folgenden allgemeinen Formel entspricht :

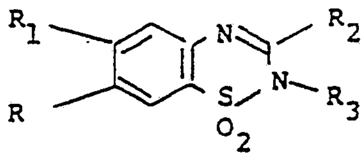

in welcher

R = Cl, $CF_3$, $SO_2$ oder $NH_2$,

$R_1$ = H, Cl, $SO_2$ oder $NH_2$,

$R_2$ = H, $C_nH_{2n+1}$ (wo n = eine ganze Zahl von 1 bis 10), $CH_2OH$, COOH oder $CH_2C_6H_5$,

$R_3$ = H, $C_nH_{2n+1}$ (wo n = eine ganze Zahl von 1 bis 10) oder $CH_2C_6H_5$,

wobei bezogen auf das Gesamtgewicht der Zusammensetzung der Anteil an (Benzo-1,2,4-thiadiazin)-1,1=Derivat 0,001 bis 10 Gew.-%, an Benzylalkohol 1,0 bis 30 Gew.-%, an Isopropylalkohol 10 bis 85 Gew.-% und an Wasser 10 bis 70 Gew.-% beträgt.

3. Eine in Lösungsform vorliegende Zusammensetzung, enthaltend (i) wenigstens ein (Benzo-1,2,4-thiadiazin)-1,1dioxyd=Derivat, (ii) wenigstens ein Benetzungsmittel, (iii) Isopropylalkohol und (iv) Wasser, wobei der pH-Wert des Systems 8,5 bis 11,0 beträgt und das (Benzo-1,2,4-thiadiazin)-1,1-dioxyd=Derivat folgender allgemeiner Formel entspricht :

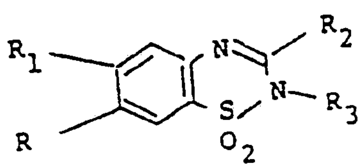

in welcher

R = Cl, $CF_3$, $SO_2$ oder $NH_2$,

$R_1$ = H, Cl, $SO_2$ oder $NH_2$,

$R_2$ = H, $C_nH_{2n+1}$ (wo n = eine ganze Zahl von 1 bis 10), $CH_2OH$, COOH oder $CH_2C_6H_5$,

$R_3$ = H, $C_nH_{2n+1}$ (wo n = eine ganze Zahl von 1 bis 10) oder $CH_2C_6H_5$,

wobei bezogen auf das Gesamtgewicht der Zusammensetzung der Anteil an (Benzo-1,2,4-thiadiazin)-1,1=Derivat 0,001 bis 10 Gew.-%, der Anteil an Benetzungsmittel 1,0 bis 50 Gew.-% und der Anteil an Wasser 10 bis 70 Gew.-% beträgt.

4. Gelöste Zusammensetzung, enthaltend (i) wenigstens ein (Benzo-1,2,4-thiadiazin)-1,1-dioxyd=Derivat, (ii) wenigstens ein Benetzungsmittel, (iii) Benzylalkohol, (iv) Isopropylalkohol und (v) Wasser, wobei der pH-Wert des Systems 8,5 bis 11,0 beträgt und das (Benzo-1,2,4-thiadiazin)-1,1-dioxyd=Derivat der folgenden allgemeinen Formel entspricht

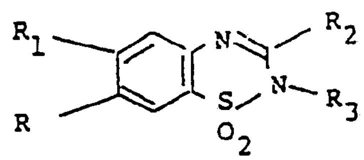

in welcher

R = Cl, $CF_3$, $SO_2$ oder $NH_2$,

$R_1$ = H, Cl, $SO_2$ oder $NH_2$,

$R_2$ = H, $C_nH_{2n+1}$ (wo n = eine ganze Zahl von 1 bis 10), $CH_2OH$, COOH oder $CH_2C_6H_5$,

$R_3$ = H, $C_nH_{2n+1}$ (wo n = eine ganze Zahl von 1 bis 10) oder $CH_2C_6H_5$,

während bezogen auf das Gesamtgewicht der Zusammensetzung der Anteil an (Benzo-1,2,4-thiadiazin)-1,1=Derivat 0,001 bis 10 Gew.-%, der Anteil an Benetzungsmittel 1,0 bis 50 Gew.-%, der Anteil an Benzylalkohol 1,0 bis 30 Gew.-%, der Anteil an Isopropylalkohol 10 bis 85 Gew.-% und der Anteil an Wasser 10 bis 70 Gew.-% beträgt.

5. Ein Mittel zur Einleitung und Förderung des Haarwuchses mit einer eine Wirksubstanz bildenden, in Form einer Lösung vorliegenden Zusammensetzung, welche (i) wenigstens ein (Benzo-1,2,4-thiadiazin)-1,1-dioxyd=Derivat, sowie (ii) Benzylalkohol enthält, wobei das (Benzo-1,2,4-thiadiazin)-1,1-dioxyd=Derivat der folgenden allgemeinen Formel entspricht :

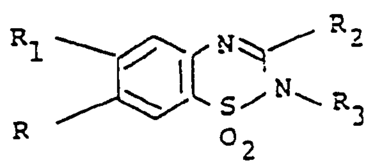

in welcher

R = Cl, $CF_3$, $SO_2$ oder $NH_2$,

$R_1$ = H, Cl, $SO_2$ oder $NH_2$,

$R_2$ = H, $C_nH_{2n+1}$ (wo n = eine ganze Zahl von 1 bis 10), $CH_2OH$, COOH oder $CH_2C_6H_5$,

$R_3$ = H, $C_nH_{2n+1}$ (wo n = eine ganze Zahl von 1 bis 10) oder $CH_2C_6H_5$,

während bezogen auf des Gesamtgewicht der Zusammensetzung der Anteil an (Benzo-1,2,4-thiadiazin)-1,1=Derivat 0,001 bis 10 Gew.-% und der Anteil an Benzylalkohol 1,0 bis 30 Gew.-% beträgt.

6. Ein Mittel zur Einleitung und Förderung des Haarwuchses mit einer eine Wirksubstanz bildenden, in Form einer Lösung vorliegenden Zusammensetzung, enthaltend (i) wenigstens ein (Benzo-1,2,4-thiadiazin)-1,1-dioxyd=Derivat, (ii) Benzylalkohol und (iii) Isopropylalkohol, sowie Wasser oder ein Gemisch derselben, wobei der pH-Wert des wasserhaltigen Systems 8,5 bis 11,0 beträgt und das (Benzo-1,2,4-thiadiazin)1,1-dioxyd=Derivat der nachstehenden allgemeinen Formel entspricht :

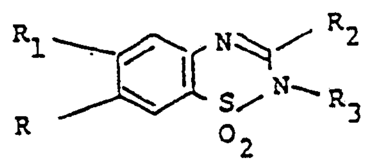

in welcher

R = Cl, $CF_3$, $SO_2$ oder $NH_2$,

$R_1$ = H, Cl, $SO_2$ oder $NH_2$,

$R_2$ = H, $C_nH_{2n+1}$ (wo n = eine ganze Zahl von 1 bis 10), $CH_2OH$, COOH oder $CH_2C_6H_5$,

$R_3$ = H, $C_nH_{2n+1}$ (wo n = eine ganze Zahl von 1 bis 10) oder $CH_2C_6H_5$,

während auf das Gesamtgewicht der Zusammensetzung bezogen der Anteil an (Benzo-1,2,4-thiadiazin)-1,1=Derivat 0,001 bis 10 Gew.-%, der Anteil an Benzylalkohol 1,0 bis 30 Gew.-%, der Anteil an Isopropylalkohol 10 bis 85 Gew.-% und der Anteil an asser 10 bis 70 Gew.-% beträgt.

7. Ein Mittel zur Einleitung und Förderung des Haarwuchses mit einer in Form einer Lösung vorliegenden und eine Wirksubstanz bildenden Zusammensetzung, welche enthält : (i) wenigstens ein (Benzo-1,2,4-thiadiazin)-1,1-dioxyd=Derivat, (ii) wenigstens ein Benetzungsmittel, (iii) Isopropylalkohol, und (iv) Wasser, wobei der pH-Wert des Systems 8,5 bis 11 beträgt und das (Benzo-1,2,4-thiadiazin)-1,1-dioxyd=Derivat der folgenden allgemeinen Formel entspricht :

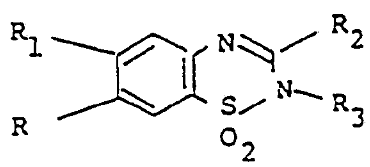

in welcher

R = Cl, $CF_3$, $SO_2$ oder $NH_2$,

$R_1$ = H, Cl, $SO_2$ oder $NH_2$,

$R_2$ = H, $C_nH_{2n+1}$ (wo n = eine ganze Zahl von 1 bis 10), $CH_2OH$, COOH oder $CH_2C_6H_5$,

$R_3$ = H, $C_nH_{2n+1}$ (wo n = eine ganze Zahl von 1 bis 10) oder $CH_2C_6H_5$,

während auf das Gesamtgewicht der Zusammensetzung bezogen der Anteil an (Benzo-1,2,4-thiadiazin)-1,1=Derivat 0,001 bis 10 Gew.-%, der Anteil an Benetzungsmittel 1 bis 50 Gew.-%, der Anteil an Isopropylalkohol 10 bis 85 Gew.-% und der Anteil an Wasser 10 bis 70 Gew.-% beträgt.

8. Ein Mittel zur Einleitung und Förderung des Haarwuchses mit einer in Form einer Lösung vorliegenden und eine Wirksubstanz bildenden Zusammensetzung, welche enthält : (i) wenigstens ein (Benzo-1,2,4-thiadiazin)-1,1-dioxyd=Derivat, (ii) wenigstens ein Benetzungsmittel, (iii) Benzylalkohol, (iv) Isopropylalkohol und (v) Wasser, wobei der pH-Wert des Systems 8,5 bis 11,0 beträgt und das (Benzo-1,2,4-thiadiazin)-1,1-dioxyd=Derivat der folgenden allgemeinen Formel entspricht :

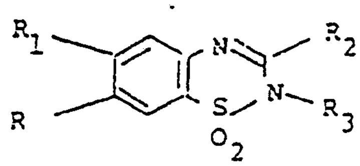

in welcher

R = Cl, $CF_3$, $SO_2$ oder $NH_2$,

$R_1$ = H, Cl, $SO_2$ oder $NH_2$,

$R_2$ = H, $C_nH_{2n+1}$ (wo n = eine ganze Zahl von 1 bis 10), $CH_2OH$, COOH oder $CH_2C_6H_5$,

$R_3$ = H, $C_nH_{2n+1}$ (wo n = eine ganze Zahl von 1 bis 10) oder $CH_2C_6H_5$,

während auf das Gesamtgewicht der Zusammensetzung bezogen der Anteil an (Benzo-1,2,4-thiadiazin)-1,1=Derivat 0,001 bis 10 Gew.-%, der Anteil an Benetzungsmittel 1,0 bis 50 Gew.-%, der Anteil an Benzylalkohol 1,0 bis 30 Gew.-%, der Anteil an Isopropylalkohol 10 bis 85 Gew.-% und der Anteil an Wasser 1,0 bis 70 Gew.-% beträgt.

**Revendications**

1. Une composition dissoute comprenant (1) au moins un dérivé (benzo-1,2,4-thiadiazine)-1,1-dioxyde et (2) de l'alcool benzylique, le dérivé (benzo-1,2,4-thiadiazine)-1,1-dioxyde ayant la formule générale :

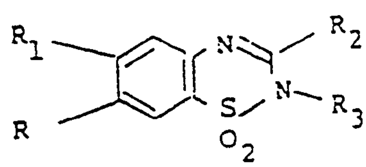

dans laquelle:

R est Cl, $CF_3$, $SO_2$ ou $NH_2$;

$R_1$ est H, Cl, $SO_2$ ou $NH_2$;

$R_2$ est H, $C_nH_{2n+1}$ dans lequel n est un nombre entier de 1 à 10, $CH_2OH$, COOH ou $CH_2C_6H_5$;

$R_3$ est H, $C_nH_{2n+1}$ dans lequel n est un nombre entier de 1 à 10, ou $CH_2C_6H_5$;

et dans laquelle la teneur en dérivé de (benzo-1,2,4-thiadiazine)-1,1- est comprise entre 0,001% et 10% en poids et la teneur en alcool benzylique est comprise entre 1,0% et 30% en poids, ces deux teneurs étant exprimées par rapport au poids total de la composition.

2. Une composition dissoute comprenant (1) au moins un dérivé (benzo-1,2,4-thiadiazine)-1,1-dioxyde, (2) de l'alcool benzylique et (3) de l'alcool isopropylique, de l'eau ou un mélange de ceux-ci, le pH du système s'il contient de l'eau étant compris entre 8,5 et 11,0, le dérivé (benzo-1,2,4-thiadiazine)-1,1-dioxyde

ayant la formule générale suivante :

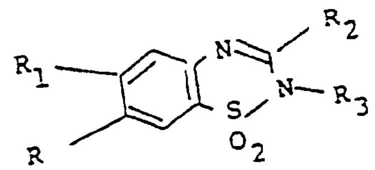

dans laquelle:

R est Cl, $CF_3$, $SO_2$ ou $NH_2$;

$R_1$ est H, Cl, $SO_2$ ou $NH_2$;

$R_2$ est H, $C_nH_{2n+1}$ dans lequel n est un nombre entier de 1 à 10, $CH_2OH$, COOH ou $CH_2C_6H_5$;

$R_3$ est H, $C_nH_{2n+1}$ dans lequel n est un nombre entier de 1 à 10, ou $CH_2C_6H_5$;

et dans laquelle la teneur en dérivé de (benzo-1,2,4-thiadiazine)-1,1- est comprise entre 0,001% et 10% en poids et la teneur en alcool benzylique est comprise entre 1,0% et 30% en poids, la teneur en alcool isopropylique est comprise entre 10% et 85% en poids, la teneur en eau est comprise entre 10% et 70% en poids, ces teneurs étant toutes exprimées par rapport au poids total de la composition.

3. Une composition dissoute comprenant (1) au moins un dérivé (benzo-1,2,4-thiadiazine)-1,1-dioxyde, (2) au moins un agent mouillant, (3) de l'alcool isopropylique, (4) de l'eau, le pH du système étant compris entre 8,5 et 11,0, le dérivé (benzo-1,2,4-thiadiazine)-1,1-dioxyde ayant la formule générale suivante :

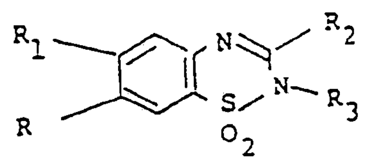

dans laquelle:

R est Cl, $CF_3$, $SO_2$ ou $NH_2$;

$R_1$ est H, Cl, $SO_2$ ou $NH_2$;

$R_2$ est H, $C_nH_{2n+1}$ dans lequel n est un nombre entier de 1 à 10, $CH_2OH$, COOH ou $CH_2C_6H_5$;

$R_3$ est H, $C_nH_{2n+1}$ dans lequel n est un nombre entier de 1 à 10, ou $CH_2C_6H_5$;

et dans laquelle la teneur en dérivé de (benzo-1,2,4-thiadiazine)-1,1- est comprise entre 0,001% et 10% en poids, la teneur en agent mouillant est comprise entre 1,0% et 50% en poids, la teneur en alcool isopropylique est comprise entre 10% et 85% en poids et la teneur en eau est comprise entre 10% et 70% en poids, ces teneurs étant toutes exprimées par rapport au poids total de la composition.

4. Une composition dissoute comprenant (1) au moins un dérivé (benzo-1,2,4-thiadiazine)-1,1-dioxyde, (2) au moins un agent mouillant, (3) de l'alcool benzylique, (4) de l'alcool isopropylique, et (5) de l'eau, le pH du système étant compris entre 8,5 et 11,0, le dérivé (benzo-1,2,4-thiadiazine)-1,1-dioxyde ayant la formule générale suivante :

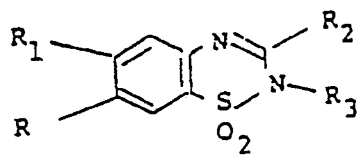

dans laquelle:

R est Cl, $CF_3$, $SO_2$ ou $NH_2$;

$R_1$ est H, Cl, $SO_2$ ou $NH_2$;

$R_2$ est H, $C_nH_{2n+1}$ dans lequel n est un nombre entier de 1 à 10, $CH_2OH$, COOH ou $CH_2C_6H_5$;

$R_3$ est H, $C_nH_{2n+1}$ dans lequel n est un nombre entier de 1 à 10, ou $CH_2C_6H_5$;

et dans laquelle la teneur en dérivé de (benzo-1,2,4-thiadiazine)-1,1- est comprise entre 0,001% et 10% en poids, la teneur en agent mouillant est comprise entre 1,0% et 50% en poids, la teneur en alcool benzylique est comprise entre 1,0% et 30% en poids, la teneur en alcool isopropylique est comprise entre 10% et 85% en poids et la teneur en eau est comprise entre 10% et 70% en poids, ces teneurs étant toutes exprimées par rapport au poids total de la composition.

5. Un agent de stimulation de la reprise et de la pousse des cheveux contenant comme principe actif, une composition dissoute comprenant (1) au moins un dérivé (benzo-1,2,4-thiadiazine)-1,1-dioxyde et (2) de l'alcool benzylique, le dérivé (benzo-1,2,4-thiadiazine)-1,1-dioxyde ayant la formule générale :

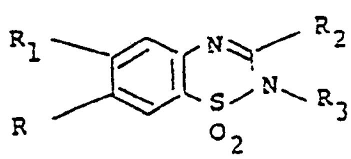

dans laquelle:

R est Cl, $CF_3$, $SO_2$ ou $NH_2$;

$R_1$ est H, Cl, $SO_2$ ou $NH_2$;

$R_2$ est H, $C_nH_{2n+1}$ dans lequel n est un nombre entier de 1 à 10, $CH_2OH$, COOH ou $CH_2C_6H_5$;

$R_3$ est H, $C_nH_{2n+1}$ dans lequel n est un nombre entier de 1 à 10, ou $CH_2C_6H_5$;

et dans laquelle la teneur en dérivé de (benzo-1,2,4-thiadiazine)-1,1- est comprise entre 0,001% et 10% en poids et la teneur en alcool benzylique est comprise entre 1,0% et 30% en poids, ces teneurs étant exprimées par rapport au poids total de la composition.

6. Un agent de stimulation de la reprise et de la pousse des cheveux contenant comme principe actif, une composition dissoute comprenant (1) au moins un dérivé (benzo-1,2,4-thiadiazine)-1,1-dioxyde, (2) de l'alcool benzylique et (3) de l'alcool isopropylique, de l'eau ou un mélange de ceux-ci, le pH du système s'il contient de l'eau étant compris entre 8,5 et 11,0, le dérivé (benzo-1,2,4-thiadiazine)-1,1-dioxyde ayant la formule générale suivante :

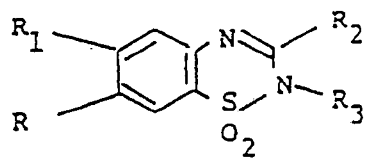

dans laquelle:

R est Cl, $CF_3$, $SO_2$ ou $NH_2$;

$R_1$ est H, Cl, $SO_2$ ou $NH_2$;

$R_2$ est H, $C_nH_{2n+1}$ dans lequel n est un nombre entier de 1 à 10, $CH_2OH$, COOH ou $CH_2C_6H_5$;

$R_3$ est H, $C_nH_{2n+1}$ dans lequel n est un nombre entier de 1 à 10, ou $CH_2C_6H_5$;

et dans laquelle la teneur en dérivé de (benzo-1,2,4-thiadiazine)-1,1- est comprise entre 0,001% et 10% en poids et la teneur en alcool benzylique est comprise entre 1,0% et 30% en poids, la teneur en alcool isopropylique est comprise entre 10% et 85% en poids et la teneur en eau est comprise entre 10% et 70% en poids, ces teneurs étant toutes exprimées par rapport au poids total de la composition.

7. Un agent de stimulation de la reprise et de la pousse des cheveux contenant comme principe actif, une composition dissoute comprenant (1) au moins un dérivé (benzo-1,2,4-thiadiazine)-1,1-dioxyde, (2) au moins un agent mouillant, (3) de l'alcool isopropylique, (4) de l'eau, le pH du système étant compris entre 8,5 et 11,0, le dérivé (benzo-1,2,4-thiadiazine)-1,1-dioxyde ayant la formule générale suivante :

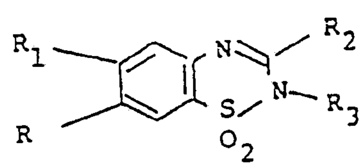

dans laquelle:

R est Cl, $CF_3$, $SO_2$ ou $NH_2$;

$R_1$ est H, Cl, $SO_2$ ou $NH_2$;

$R_2$ est H, $C_nH_{2n+1}$ dans lequel n est un nombre entier de 1 à 10, $CH_2OH$, COOH ou $CH_2C_6H_5$;

$R_3$ est H, $C_nH_{2n+1}$ dans lequel n est un nombre entier de 1 à 10, ou $CH_2C_6H_5$;

et dans laquelle la teneur en dérivé de (benzo-1,2,4-thiadiazine)-1,1- est comprise entre 0,001% et 10% en poids, la teneur en agent mouillant est comprise entre 1,0% et 50% en poids, la teneur en alcool isopropylique est comprise entre 10% et 85% en poids et la teneur en eau est comprise entre 10% et 70% en poids, ces teneurs étant toutes exprimées par rapport au poids total de la composition.

8. Un agent de stimulation de la reprise et de la pousse des cheveux contenant comme principe actif, une composition dissoute comprenant (1) au moins un dérivé (benzo-1,2,4-thiadiazine)-1,1-dioxyde, (2) au moins un agent mouillant, (3) de l'alcool benzylique, (4) de l'alcool isopropylique, et (5) de l'eau, le pH du système étant compris entre 8,5 et 11,0, le dérivé (benzo-1,2,4-thiadiazine)-1,1-dioxyde ayant la formule générale suivante :

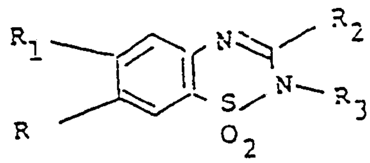

dans laquelle:

R est Cl, $CF_3$, $SO_2$ ou $NH_2$;

$R_1$ est H, Cl, $SO_2$ ou $NH_2$;

$R_2$ est H, $C_nH_{2n+1}$ dans lequel n est un nombre entier de 1 à 10, $CH_2OH$, COOH ou $CH_2C_6H_5$;

$R_3$ est H, $C_nH_{2n+1}$ dans lequel n est un nombre entier de 1 à 10, ou $CH_2C_6H_5$;

et dans laquelle la teneur en dérivé de (benzo-1,2,4-thiadiazine)-1,1- est comprise entre 0,001% et 10% en poids, la teneur en agent mouillant est comprise entre 1,0% et 50% en poids, la teneur en alcool benzylique est comprise entre 1,0 et 30% en poids, la teneur en alcool isopropylique est comprise entre 10% et 85% en poids et la teneur en eau est comprise entre 1,0% et 70% en poids, ces teneurs étant toutes exprimées par rapport au poids total de la composition.